Europäisches Patentamt

European Patent Office

Office européen des brevets

(11) Publication number: **0 071 058**
**B1**

(12) **EUROPEAN PATENT SPECIFICATION**

(45) Date of publication of patent specification: **11.11.87**

(21) Application number: **82106100.9**

(22) Date of filing: **08.07.82**

(51) Int. Cl.⁴: **A 61 B 1/12, B 08 B 9/02**

(54) **Washing device of endoscope fluid pipes.**

(30) Priority: **17.07.81 JP 111940/81**

(43) Date of publication of application:
**09.02.83 Bulletin 83/06**

(45) Publication of the grant of the patent:
**11.11.87 Bulletin 87/46**

(84) Designated Contracting States:
**DE**

(56) References cited:
**DE-A-2 908 612**
**FR-A- 727 613**
**FR-A-2 462 897**
**US-A-3 963 438**

(73) Proprietor: **OLYMPUS OPTICAL CO., LTD.**
**43-2, 2-chome, Hatagaya Shibuya-ku**
**Tokyo 151 (JP)**

(72) Inventor: **Yamaguchi, Tatuya**
**106 Hinohirayamadai-jutaku 6-7-8, Asahigaoka**
**Hino-shi Tokyo (JP)**

(74) Representative: **Kempe, Wolfgang, Dr.**
**Postfach 1273**
**D-6800 Mannheim 1 (DE)**

## Description

This invention relates to a washing device of endoscope fluid pipes. An endoscope generally comprises various pipes through which air, water and other liquids are conducted. After, therefore, the application of an endoscope, it is necessary to thoroughly wash not only the peripheral surface of the endoscope but also the interior of the liquid pipes. The conventional process of washing the interior of the liquid pipes has the drawbacks that a washing liquid-pouring pipe has to be attached to the mouthpieces of the respective fluid pipes; the operation of the valves of the fluid pipes by their control valves is accompanied with troublesome work; and the detailed parts of the control valves and those parts of a cylinder which are covered with a piston tends to washed very imperfectly.

It is accordingly the object of this invention to provide a washing device of endoscope fluid pipes which allows for the easy fitting of a washing liquid-feeding device to the respective endoscope fluid pipes and effects the reliable washing of the interior of the fluid pipes and their valve devices.

To attain the above-mentioned object, this invention provides a washing device of a pipe of an endoscope comprising at least one valve for controlling the operation of said pipe said valve being formed of a cylinder which is detachably fitted with a valve member leaving an opening exposed to the outside when said valve member is removed, the said washing device characterized by comprising at least one connector which, when the valve member is removed, communicates with the opening of the cylinder in liquid tightness and which is provided with an inlet port communicating with the interior of said cylinder and a washing liquid-feeding tube which is connected to the connector and communicates with the inlet port whereby a washing liquid is conducted into the cylinder and the pipe communicating therewith through said tube and inlet port.

This invention can be more fully understood from the following detailed description when taken in conjunction with the accompanying drawings, in which:

Fig. 1 shows the arrangement of an endoscope adapted to be fitted with a fluid pipe-washing device according to a first embodiment of this invention;

Fig. 2 indicates the arrangement of a water-storage tank;

Fig. 3 is a lateral sectional view of a valve device of an endoscope fluid pipe;

Fig. 4 illustrates the manner in which the fluid pipe-washing device according to the first embodiment of the invention is applied to the endoscope shown in Fig. 1;

Fig. 5 is a lateral sectional view of the connector of the fluid pipe-washing device according to the first embodiment of the invention when connected to the endoscope shown in Fig. 1;

Fig. 6 sets forth the arrangement of an endoscope adapted to be fitted with a fluid pipe-washing device according to a second embodiment of the invention; and

Fig. 7 is a sectional view of an endoscope fluid pipe-washing device according to a third embodiment of the invention.

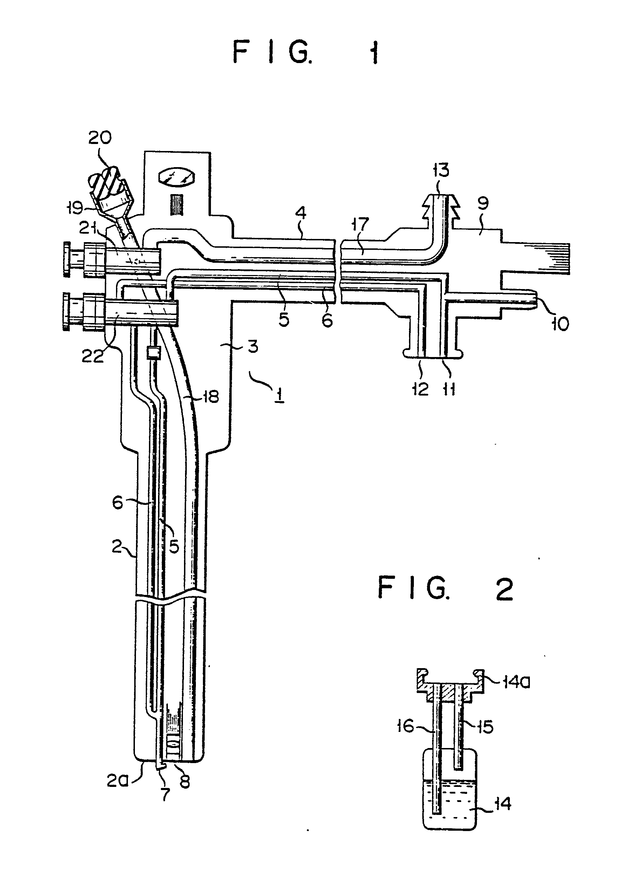

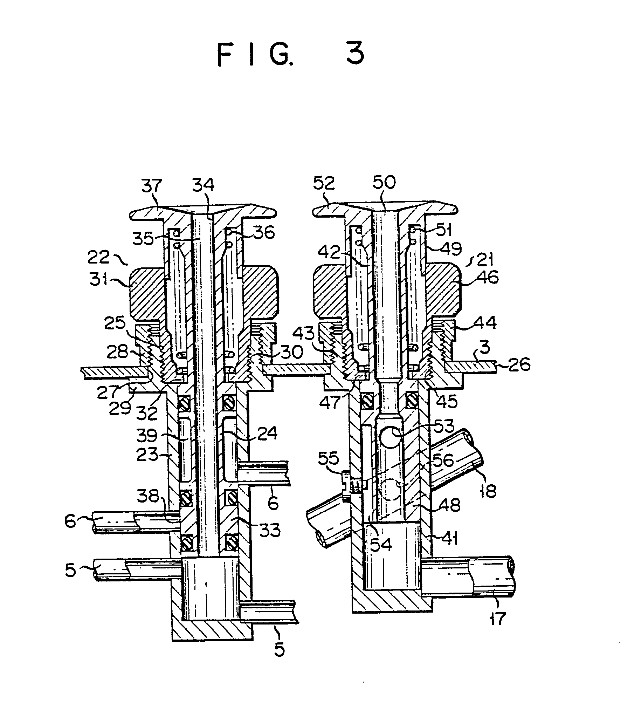

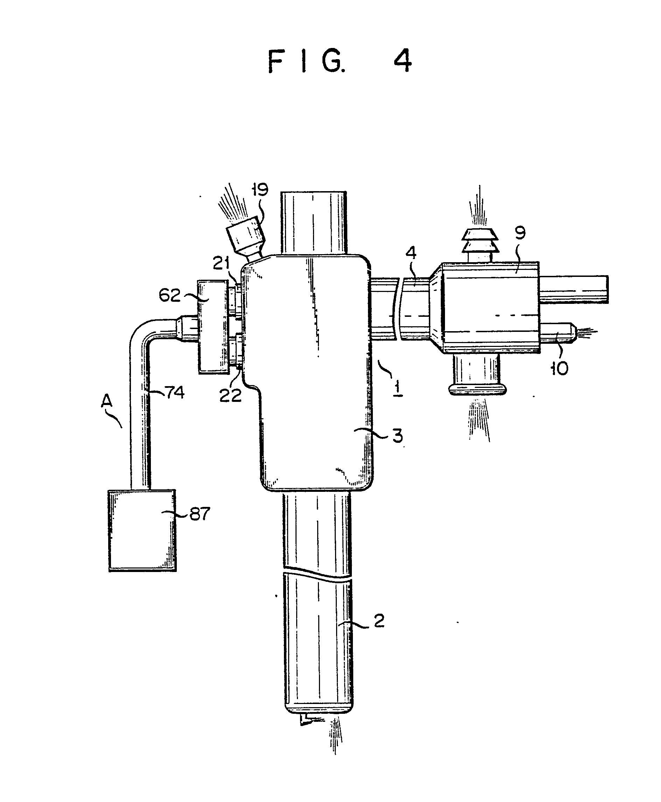

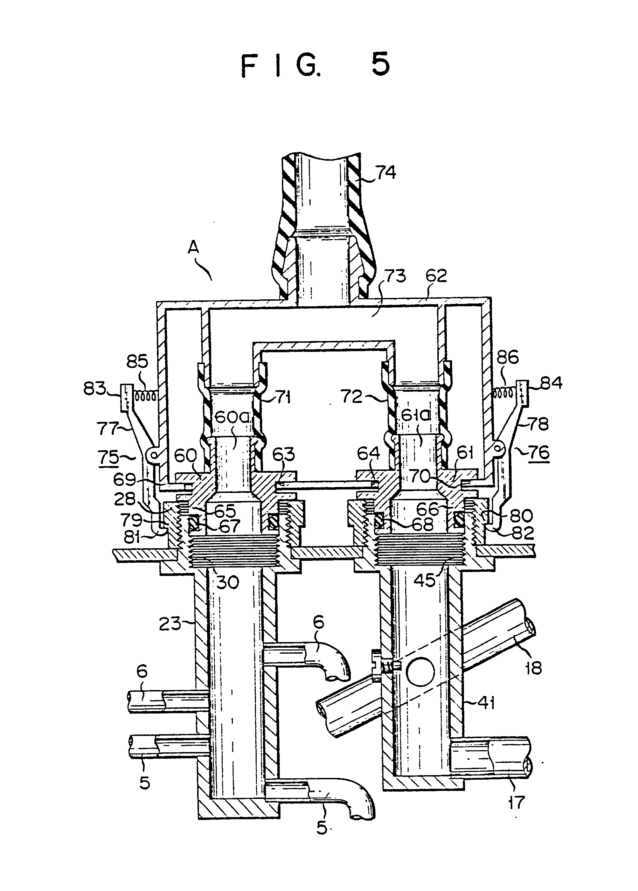

Description is now given with reference to Figs. 1 to 5 of an endoscope fluid pipe-washing device according to a first embodiment of the invention.

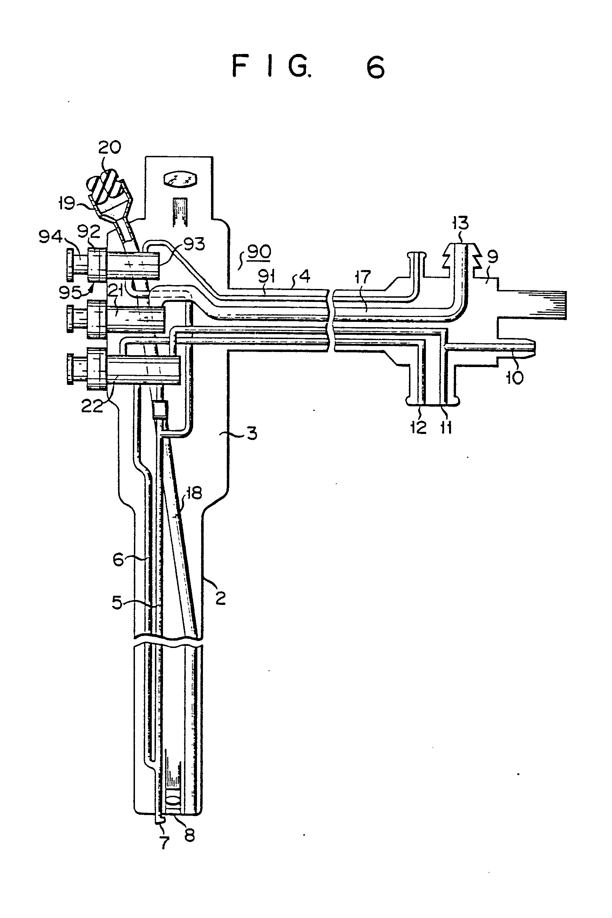

Reference numeral 1 denotes an endoscope, which comprises an insertion section 2, control section 3, light guide cable 4 and connector 9. Fluid pipes to be described later extend through the endoscope 1. An air pipe 5 and water pipe 6 extend through the insertion section 2, control section 3 and light guide cable 4. The converged downstream ends of the air pipe 5 and water pipe 6 are connected to an air-water ejecting nozzle 7 fitted to the distal end of the insertion section 2. The tip of the air-water ejecting nozzle 7 is directed toward the outer surface of an observation window 8. The nozzle 7 ejects water or air to the outer surface of the observation window 8 for cleaning. A connector 9 is fitted to the extended end of the light guide cable 4. The connector 9 comprises first and second air-ejecting mouthpieces 10, 11 communicating with the air pipe 5, a water-ejecting mouthpiece 12 communicating with the water pipe 6 and a suction mouthpiece 13 communicating with a suction pipe to be described later. When the connector 9 is fitted to a light source device (not shown), the first air-injecting mouthpiece 10 is connected to an air pump held in a light source device. The second air-ejecting mouthpiece 11 and water-ejecting mouthpiece 12 are respectively connected to an air-ejecting pipe 15 and water-ejecting pipe 16 attached to the water storage tank 14 (Fig. 2). Reference numeral 14a denotes an additional connector detachably fitted to the connector 9. Said additional connector 14a is used when the air-ejecting pipe 15 and water-ejecting pipe 16 fitted to the water storage tank 14 are respectively connected to the aforesaid second air-ejecting mouthpiece 11 and water-ejecting mouthpiece 12. The suction mouthpiece 13 is connected to a suction device (not shown). A suction pipe 17 extends through the insertion section 2, control section 3 and light guide cable 4. A treating implement-guiding channel 18 is concurrently used as the distal end portion of the suction pipe 17. The distal end of the treating implement-guiding channel 18 is open at the distal end 2a of the insertion section 1. The proximal end of said treating implement-guiding channel 18 constitutes a port 19 open to the outside to allow for the insertion of treating implements. A forceps plug 20 is detachably fitted to the port 19. The forceps plug 20 is formed of, for example, an elastic ring to allow for the insertion of a treating implement such as forceps into the treating implement-guiding channel 18.

The channel 18 is connected to the proximal end of the suction pipe 17 by means of a suction valve 21. An air-water valve 22 is set in the intermediate part of the air pipe 5 and water pipe 6. The suction valve 21 and air-water valve 22 are arranged in parallel in the proximity of the lateral wall of the control section 3 as shown in Fig. 3. The air-water valve 22 consists of a blind cylinder 23 acting as a valve seat and a hollow piston 24 acting as a valve member. The open end portion 25 of the cylinder 23 has larger inner and outer diameters than any other part of the cylinder 23. The open end portion 25 is tightly inserted into a penetrating hole 27 bored in the wall 26 of the control section 3. When a set ring 28 is threadedly fitted around the peripheral wall of the outer end portion of the cylinder 23, the wall 26 is securely clamped between the set ring 28 and the flange 29. A threaded cylindrical fitting ring 31 is detachably engaged with the outer periphery of a larger diameter section 30 formed on the inside of the open end portion 25. The engaged end portion of the fitting ring 31 is provided with a flange 32 projecting into the ring 31. The piston 24 acting as a valve consists of a larger diameter inner end portion 33 and a smaller diameter outer end portion 34. The inner end portion 33 slides through the cylinder 23. A release hole 35 extends axially through the piston 24. The other end portion 34 of the piston 24 projects from the cylinder 23. A compression coil spring 36 is wound about the outer end portion 34 of the piston 34. This compression coil spring 36 is interposed between the flange 32 and a finger plate formed at the outer end of the piston 24 to urge the piston 24 outward. The piston 24 is normally kept in a projecting waiting position with the end face of the inner section 33 pressed against the flange 32. When the fitting ring 31 is taken off, the piston 24 can be easily pulled out of the cylinder 23. An air-water pipe-closing peripheral wall 38 is formed in the inner end region of the inner section 33 of the piston 24. An annular communication groove 39 is formed in the peripheral wall of the outer end region of the inner section 33 of the piston 24.

In the cylinder 23, the influx end of the water pipe 6 is open to the communication groove 39 of the piston 24 when kept in a waiting position. The efflux end of said water pipe 6 is open to the pipe-closing peripheral wall 38. When the piston 24 is pushed in, the annular communication groove 39 is set between the influx end and efflux end of the water pipe 6. The influx end of the air pipe 5 is open to the bottom of the cylinder 23. The efflux end of the air pipe 5 is positioned in the intermediate part of the cylinder 23. When the piston 24 is pushed in, the efflux end of the air pipe 5 is closed by the aforesaid pipe-closing peripheral wall 38 of the piston 24. While the piston 24 remains in a waiting position, the water pipe 5 is shut off by the pipe-closing peripheral wall 38. At this time, the air pipe 5 is left open and communicates with the outside through the release hole 35. Therefore, all air streams are drawn off to the outside, making it impossible to introduce air streams. Namely, while the piston 24 remains in a waiting position, neither air streams nor water is supplied. When air streams are supplied, it is advised to put the underside of the finger tip on the finger plate 38 thereby to close the release hole 35 and stop the release of air streams. When water is introduced, it is advised to push in the piston while the release hole 35 is closed with the finger top thereby to shut off the air pipe 5 and open the water pipe 6 by means of the annular communication groove 39.

Like the air-water valve 22, the suction valve 21 comprises a cylinder 41, piston 42, open end section 43 of the piston 42, set ring 44, larger diameter hole 45, fitting ring 46, flange 47, inner end region 48, outer end region 49, release hole 50, compression coil spring 51 and finger plate 52. Formed in the inner end region 48 of the piston 42 are an air hole 53 and axially extending guide groove 54. A pin 55 is inserted into the guide groove 54 to stop the rotation of the piston 42. The suction end of the suction pipe 17 is connected to the bottom of the cylinder 23. A hole 56 communicating with a treating implement-guiding channel 18 is provided at a point which faces the air hole 53 when the piston 42 is pushed in. While the piston 42 remains in a waiting position, the communication hole 56 is closed by the peripheral wall of the inner end region 48 of the piston 42. At this time, external air streams alone are sucked in through the release hole 50, but not through the treating implement guiding channel 18. When the piston 42 is pushed in with the release hole 50 closed with the finger, then the air hole 53 and communication hole 56 are aligned with each other, assuring the suction of air streams through the suction pipe 17.

Description is now given of a washing device A (Figs. 4 and 5) used to clean the various pipes 5, 6, 17 of the endoscope 1. The washing device A comprises a pair of connectors 60, 61, which are respectively fitted into the openings of the cylinders 23, 41 in liquid tightness, after removing the assembly of the coil spring 36 and piston 24 and the assembly of the coil spring 51 and piston 42. The connectors 60, 61 are substantially cylindrical and respectively provided with air-water inlet ports 60a, 61a. The connectors 60, 61 are fitted into openings 63, 64 formed in the front portion of a box-shaped socket body 62. Ring-shaped elastic packings 67, 68 respectively engageable with the larger diameter sections 30, 45 of the cylinders 23, 41 are fitted around the peripheries of the engagement sections 65, 66 of the connectors 60, 61 which project from the front part of the socket body 62. The peripheral edges of the openings 63, 64 are respectively loosely fitted into the annular engagement grooves 69, 70 formed in the peripheral walls of the connectors 60, 61, thereby enabling the relative positions of the connectors 60, 61 to be varied, and consequently fully absorbing a slight displacement occurring between the cylinders 23, 41 from the various factors involved in their manufacture and their different types. The connectors 60, 61 are connected to a connection pipe 73 provided in the socket body 62 by means of the corresponding pliant tubes 71, 72. The connectors 60, 61 communicate through the connection pipe 73 with a liquid-conducting tube 74 extending out of the connection pipe 73. The lateral wall of the socket body 62 is provided with members 75, 76 which cause the socket body 62 to be engaged with the endoscope 1 when the connectors 60, 61 are fitted into the cylinders 23, 41. The engagement members 75, 76 respectively comprise engagement levers 77, 78 whose intermediate parts are swingably fitted to the lateral wall of the socket body 62. The engagement levers 77, 78 act at one end as engagement pawls 81, 82 engageable with the stepped engagement portions of the set rings 28, 44, and are used at the other end as control sections 83, 84. Compression coil springs 85, 86 are respectively interposed between the lateral wall of the socket body 62 and the control sections 83, 84 to urge the engagement levers 77, 78, thereby causing the engagement pawls 81, 82 to be engaged with the stepped engagement sections 79, 80 of the set rings 28, 44. The liquid-conducting tube 74 is connected to a washing liquid feeder 87 (Fig. 4). Where the washing device A is applied, the pistons 24, 42, fitting rings 31, 46 and compression coil springs 36, 51 are removed from the valves 21, 22 to open the cylinders 23, 41 at one end (Fig. 5). The connectors 60, 61 are respectively inserted into the openings of the cylinders 23, 41, and the socket body 62 is pushed in. At this time, the pawls 81, 82 of the engagement levers 77, 78 respectively slide over the outer peripheral wall of the set rings 28, 44 and are engaged with the stepped engagement sections 79, 80, thereby causing the socket body 62 to be securely set in place. A pressurized washing liquid (rinsing water, detergent solution, or sterilizing liquid) is supplied from a washing liquid source 87 through the liquid-conducting tube 74. The washing liquid is diverted in two directions in the connection pipe 73. One diversion of the washing liquid flows into the cylinder 23 of the air-water valve 22 through the inlet port 60a of the connector 60, and the pipes 5, 6 communicating with the cylinder 23, and is drawn out through the air-water nozzle 7, air-ejecting mouthpieces 10, 11 and water-ejecting mouthpiece 12. As a result, it is possible to wash the interior of the cylinder 23 and the pipes 5, 6 communicating with the cylinder 23. The other diversion of the washing liquid which flows into the other connector 61 through its inlet port 61a runs into the suction pipe 17 and treating implement-guiding channel 18 which are made to communicate with the washed cylinder 41 of the suction valve 22, and is drawn off at the opposite ends of the pipe 17 and channel 18. Consequently it is possible to wash the interior of the cylinder 41, suction pipe 17 and treating implement-guiding channel 18. The forceps plug 20, pistons 24, 42 and fitting rings 31, 46 taken off the endoscope 1 are individually cleaned, for example, by a brush. With foregoing embodiment, the connectors 60, 61 are respectively engaged with the cylinders 23, 41, making it possible to clean the interior of the cylinders 23, 41 and pipes 5, 6, 7 at the same time.

It is possible to provide the connectors 60, 61 of the foregoing embodiment separately and attach individual engagement means thereto, and fix the connectors 60, 61 respectively to the cylinders 23, 41.

Description is now given with reference to Fig. 6 of the arrangement of an endoscope 90 adapted to be fitted with a fluid pipe-washing device according to a second embodiment of the invention. This endoscope 90 is constructed by inserting a gas pipe 91 for supplying an uninflammable gas such as carbon dioxide into the control section 3 of the endoscope 1 of the foregoing embodiment and inserting a gas valve 92 into the gas pipe 91 in parallel with the valves 21, 22. Like the valves 21, 22, this gas valve 92 consists of a cylinder 93 and piston 94. The piston 94 is detachably fitted to the cylinder 93 by means of a fitting ring 95.

The washing device of the endoscope 90 is constructed by providing similar connectors to the connectors 60, 61 of the preceding embodiment in parallel relationship in such a state as is insertible in water tightness into the opening of the cylinder 93 stripped of the piston 94. In other words, the second embodiment of Fig. 6 comprises three connectors which can be respectively fitted into the cylinders 23, 41, 93 of the valves 21, 22, 92 at the same time.

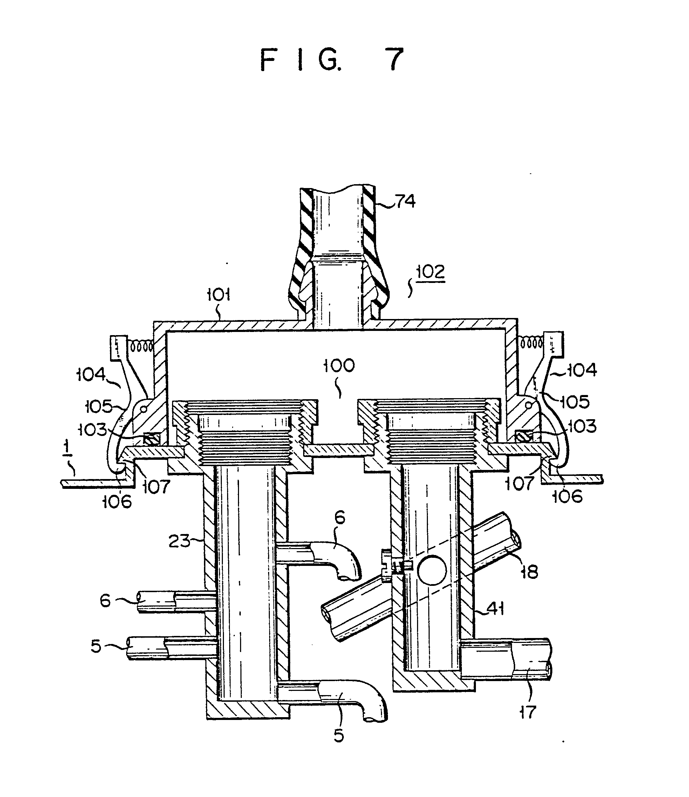

Description is now given with reference to Fig. 7 of an endoscope fluid pipe-washing device according to a third embodiment of this invention. With this embodiment, an individual connector is not fitted into the openings of the cylinders 23, 41. Instead, a connector 102 is formed of a box-shaped socket body 101 which comprises an inlet port 100 communicating with the openings of the cylinders 23, 41. The peripheral edge of the opening of the socket body 101 is fitted with a packing 103 which is pressed against the surface of the control section 3 of the endoscope 1, thereby enabling the socket body 101 to be connected to the control section 3 in liquid tightness. The engagement pawls 106 of the engagement levers 105 acting as engagement means 104 constructed substantially in the same manner as in the foregoing embodiments are made engageable with engagement projections 107 provided on the periphery of the cylinders 23, 41.

With the aforementioned endoscope fluid pipe-washing device of this invention, the valves of the endoscope pipes are removed to open the corresponding cylinders. The connectors are fitted into said openings to supply a washing liquid from its source, and a washing liquid is introduced into the cylinders and the pipes communicating with said cylinders for washing. In other words, only the fitting of the connectors into the openings of the corresponding cylinders allows for the easy washing of the interior of the cylinders and the endoscope pipes communicating with the cylinders, making it unnecessary to control the pipe valves and simplifying the washing operation.

Further, washing is carried out by pouring a washing liquid into a cylinder from which the valve body is removed, thereby assuring uniform thorough washing.

## Claims

1. A washing device of a pipe (5, 6, 91, 71, 18) of an endoscope (1, 90) comprising at least one valve (21, 22, 92) for controlling the operation of said pipe (5, 6, 91, 17, 18), said valve (21, 22, 92) being formed of a cylinder (23, 93, 41) which is detachably fitted with a valve member (24, 42, 94) leaving an opening exposed to the outside when said valve member (24, 42, 94) is removed, the said washing device characterized by comprising at least one connector (60, 61, 102) which, when the valve member (24, 42, 94) is removed, communicates with the opening of the cylinder (23, 93) in liquid tightness and which is provided with an inlet port (60a, 61a, 100) communicating with the interior of said cylinder (23, 41, 93); and a washing liquid-feeding tube (74) which is connected to the connector (60, 61, 102) and communicates with the inlet port (60a, 61a, 102) whereby a washing liquid is conducted into the cylinder (23, 41, 93) and the pipe (5, 6, 17, 18, 91) communicating therewith through said tube (74) and inlet port (60a, 61a, 100).

2. The endoscope pipe-washing device according to claim 1, wherein the inlet port (100) communicates directly in liquid tightness with more than one cylinder's opening (23, 41, 93 Fig. 7) of said valves (21, 22, 92) from which the valve member (24, 42, 94) has been removed.

3. The endoscope pipe-washing device according to claim 1 or 2, wherein said washing device further comprises engagement means (75, 76, 104) which is engaged with the endoscope (1, 90) to hold the device when the connector (60, 61, 102) is fitted onto the endoscope (1, 90).

## Patentansprüche

1. Waschvorrichtung für ein Rohr (5, 6, 17, 18, 91) eines Endoskopes (1, 90) mit mindestens einem Ventil (21, 22, 92) zur Steuerung der Zuschaltung des Rohres, wobei das Ventil aus einem Zylinder (23, 41, 93) besteht, der abnehmbar auf einem Ventilglied (24, 42, 94) sitzt und eine nach außen gerichtete Öffnung freigibt, wenn das Ventilglied entfernt wird, dadurch gekennzeichnet, daß wenigstens ein Verbindungsstück (60, 61, 102) vorgesehen ist, das, wenn das Ventilglied entfernt ist, mit der Öffnung des Zylinders in flüssigkeitsdichter Verbindung steht und das eine Einlaßöffnung (60a, 61a, 100) aufweist, die mit dem Innenraum des Zylinders in Verbindung steht, und daß ein Waschflüssigkeits-Zuführschlauch (74) vorgesehen ist, der mit dem Verbindungsstück verbunden ist und mit der Einlaßöffnung in Verbindung steht, wodurch eine Waschflüssigkeit in den Zylinder und die Rohre geleitet wird, die demzufolge mit dem Schlauch und der Einlaßöffnung verbunden sind.

2. Waschvorrichtung nach Anspruch 1, bei der die Einlaßöffnung (100) mit mehr als eines Zylinders Öffnung (23, 41, 93, Figur 7) des Ventiles (21, 22, 92), von dem das Ventilglied entfernt ist, in flüssigkeitsdichter Verbindung steht.

3. Waschvorrichtung nach Anspruch 1 oder 2, bei der ferner Verbindungsmittel (75, 76, 104) vorgesehen sind, die mit dem Endoskop (1, 90) in Eingriff stehen, um die Waschvorrichtung zu halten, wenn das Verbindungsstück (60, 61, 102) auf das Endoskop aufgesetzt ist.

## Revendications

1. Dispositif de lavage pour un tuyau (5, 6, 91, 17, 18) d'un endoscope (1, 90) comprenant au moins une valve (21, 22, 92) commandant le fonctionnement dudit tuyau (5, 6, 91, 17, 18), ladite valve (21, 22, 92) étant formée d'un cylindre (23, 93, 41) qui est monté de façon amovible à un élément (24, 42, 94) de valve laissant une ouverture découverte vis-à-vis de l'extérieur lorsque cet élément de valve (24, 42, 94) a été enlevé, ce dispositif de lavage étant caractérisé par le fait qu'il comprend au moins un connecteur (60, 61, 102) qui, lorsque l'élément de valve (24, 42, 94) a été enlevé, communique avec l'ouverture du cylindre (23, 93) de façon étanche vis-à-vis des liquides et qui est pourvu d'un orifice d'entrée (60a, 61a, 100) communiquant avec l'intérieur dudit cylindre (23, 41, 93); et un tube (74) d'alimentation en liquide de lavage qui est relié au connecteur (60, 61, 102) et qui communique avec l'orifice d'entrée (60a, 61a, 102), grâce à quoi le liquide de lavage est dirigé dans le cylindre (23, 41, 93) et dans le tuyau (5, 6, 17, 18, 91) communiquant avec ce dernier par l'intermédiaire du tube (74) et de l'orifice d'entrée (60a, 61a, 100).

2. Dispositif de lavage de tuyau d'endoscope selon la revendication 1, dans lequel l'orifice d'entrée (100) communique directement de façon étanche vis-à-vis des liquides avec plusieurs ouvertures (23, 41, 93, Fig. 7) de cylindre des valves (21, 22, 92) dont a été enlevé l'élément (24, 42, 94) de valve.

3. Dispositif de lavage de tuyau d'endoscope selon la revendication 1 ou 2, dans lequel le dispositif de lavage comprend en outre, des moyens d'engagement (75, 76, 104) qui coopèrent avec l'endoscope (1, 90) pour supporter le dispositif quand le connecteur (60, 61, 102) est monté sur l'endoscope (1, 90).

# F I G. 1

# F I G. 2

# F I G. 3

# F I G. 4

# F I G. 5

# F I G. 6

# F I G. 7